# EUROPEAN PATENT SPECIFICATION

(11) **EP 1 509 491 B1**
(45) Date of publication and mention of the grant of the patent: **20.09.2006**
(21) Application number: 03725965.2
(22) Date of filing: 22.05.2003
(51) Int. Cl.: C07C 225/16, C07C 229/34

(54) **INTERMEDIATE PRODUCTS, METHODS FOR THEIR PREPARATION AND USE THEREOF**
ZWISCHENPRODUKTE, VERFAHREN FÜR DEREN HERSTELLUNG UND DEREN GEBRAUCH
PRODUITS INTERMEDIAIRES, PROCEDES PERMETTANT DE LES PREPARER ET LEUR UTILISATION

(30) Priority: 31.05.2002 EP 02445069
(43) Date of publication of application: 02.03.2005
(73) Proprietor: Personal Chemistry i Uppsala AB, 753 18 Uppsala (SE)
(72) Inventor: WESTMAN, Jacob, S-740 20 Vänge (SE); LUNDIN, Ronny, S-178 32 Ekerö (SE)
(74) Representative: Halldin, Bo
(86) International application number: PCT/SE2003/000827
(87) International publication number: WO 2003/101933

(56) References cited:
- WO-A-99/38993
- BERTEINA S ET AL: "RADICAL AND PALLADIUM-MEDIATED CYCLIZATIONS OF ORTHO-IODO BENZYL ENAMINES: APPLICATION TO SOLID PHASE SYNTHESIS" SYNLETT, THIEME VERLAG, STUTTGART, DE, November 1998 (1998-11), pages 1231-1233, XP001074013 ISSN: 0936-5214
- STANOVNIK B. ET AL.: "ALKYL 2-SUBSTITUTED 3-(DIMETHYLAMINO)PROPENOATES AND RELATED COMPOUNDS - VERSATILE REAGENTS IN HETEROCYCLIC CHEMISTRY" SYNLETT, 2000, pages 1077-1091, XP002219156 cited in the application
- LARHED M ET AL: "MICROWAVE-ASSISTED HIGH-SPEED CHEMISTRY: A NEW TECHNIQUE IN DRUG DISCOVERY" DRUG DISCOVERY TODAY, ELSEVIER SCIENCE LTD, GB, vol. 6, no. 8, April 2001 (2001-04), pages 406-416, XP002901866 ISSN: 1359-6446 cited in the application

## Description

The present invention relates to novel solid supported intermediate products suitable for synthesis of heterocyclic compounds and methods for preparing such intermediate products. The invention also relates to use of the intermediate products in simple and fast methods on solid phase for synthesis of heterocycles with large structural diversity in high yields and high purity.

Highly functionalized heterocycles of various ring sizes, with different heteroatoms and substitution patterns are of major interest in the pharmaceutical and agricultural industry due to the many intrinsic biological properties of these substances.

In medicinal chemistry in general, and combinatorial chemistry in particular, the use of versatile synthons or versatile scaffolds, which are available after only a few reaction steps, are of great interest. An example of a reagent producing such synthons is N,N-dimethylformamide diethyl acetal (DMFDEA), cf. Abdulla, R.F.; Brinkmeyer, R.V., *Tetrahedron,* **1979**, 35, 1675-1735. Condensation reactions between an activated methyl or methylene group adjacent to an ester or keto functionality and DMFDEA form dimethylaminopropenoates(A) or dimethylaminopropenones (B), see Fig. 1.

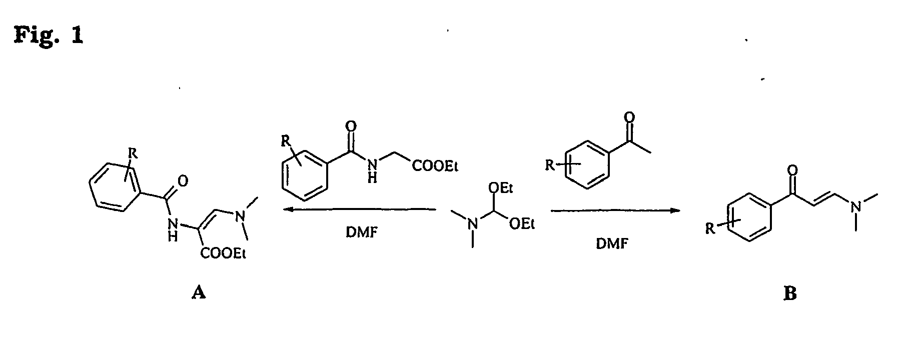

These intermediates, in which the dimethylamino moiety acts as a good leaving group, have been used in reactions in solution under conventional heating methods which has been described for example in Stanovnik, B.; Svete, J., *Synlett,* **2000,** *8*, 1077-1091. The intermediates could then be reacted with dinucleophiles to form different heterocycles. The availability of starting materials, which could form activated alkylaminopropenones or alkylaminopropenoates with DMFDEA is large and the number of possible heterocycles with large diversity, which are possible to form in a subsequential step from these types of intermediates is substantial. The formation of heterocycles from these intermediates takes place via a cascade or domino-type reaction, cf. Tietze, L.F., *Chem.Rev.,* **1996***,* 115-136, which means that it involves two or more new bond formations taking place under the same reaction conditions. The advantages of this kind of reaction as compared to traditional multi-step reactions are simplified engineering, no intermediate work-up, minimized waste handling and lower cost of purification. All of these are important factors to consider when working the synthesis of combinatorial libraries.

Microwave heating has been used in organic synthesis since 1986, cf. Gedye, R.; Smith, F.; Westaway, K.; Ali, H.; Baldisera, L.; Laberge, L.; Rousell, J., *Tetrahedron Lett.* **1986**, *27*, 279-282. Microwave heating reduces the reaction times in comparison with traditional heating. In addition, the yields of the reactions are often increased and the time for optimizing the reaction conditions is minimized in comparison to conventional heating methods.

The present invention relates in one aspect to new intermediate products of the general formula I as defined in claim 1 suitable for synthesis of heterocyclic compounds.

In another aspect the present invention relates to a method for preparing intermediates of formula I as defined in claim 2. In a further aspect the invention relates to a method for preparing intermediates of formula I as defined in claim 3.

According to another aspect the invention relates to the use of an intermediate product of the general formula I for synthesis of heterocyclic compounds as defined in claim 4.

More closely the invention relates to an intermediate product suitable for synthesis of heterocyclic compounds which intermediate compound has the general formula I 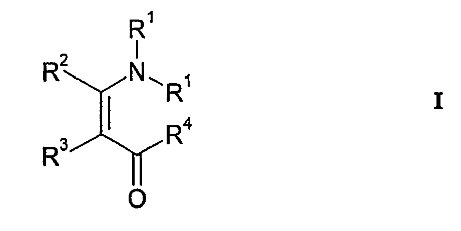 coupled to a solid polymeric support through one or both of the R¹ group(s) or through the R⁴ group
*wherein when coupled to the polymeric support through R*^{*1*}

The R¹ groups represent the same or different groups chosen from lower alkyl with 1 to 6 carbon atoms, such as methyl, ethyl, cycloalkyl with 3 to 6 carbon atoms in the ring, such as cyclopentyl, cyclohexyl, heterocyclic compounds including one or more heteroatoms, benzyl groups. Two R¹ groups together can be included in a heterocyclic ring containing one or more nitrogen atoms ;
R² represents H or a lower alkyl with 1 to 6 carbon atoms, such as methyl;
R⁴ represents unsubstituted or substituted aromatic ring(s), unsubstituted or substituted heteroaromatic ring(s) with one or more heteroatoms, or OR⁵; when R⁴ is unsubstituted or substituted aromatic ring(s), unsubstituted or substituted heteroaromatic ring(s), R³ represents H, alkyl, unsubstituted or substituted aromatic ring, unsubstituted or substituted heteroaromatic ring with one or more heteroatoms, or COOR⁵;
when R⁴ is OR⁵, R³ is CN, COOR⁵, NCOR⁵, NCOOR⁵, or COR⁵;
the R⁵ groups, which can be the same or different, represent H, alkyl, benzyl, unsubstituted or substituted aromatic ring(s), unsubstituted or substituted heteroaromatic ring(s) with one or more heteroatoms;
*and wherein when coupled to the polymeric support through R*^{*4*}
R¹ and R² are as defined above,
R³ is as defined above except COOR⁵ and COR⁵,
R⁴=OR⁵ where R⁵ is as defined above except H.

When producing a large number of substances in solution the subsequent purifications will be time-consuming. However, it has according to the present invention been found that solid phase technique, which allows for easy automation together with microwave assisted heating, gives rise to unexpected advantages such as high yields of the heterocyclic compounds with high purity and possibility to eliminate time-consuming purification steps, which are necessary when producing heterocyclic compounds in solution according to the above mentioned prior art. Furthermore, in the method according to the present invention a large excess of reagent could be used in a reaction with a resin bound substrate whereby the reaction is driven to completion. The redundant amount of reagent could then be removed by a simple filtration. Furthermore, by the use of microwave heating in solid phase synthesis in accordance with the present invention the reaction times in the heterogeneous systems can be substantially shortened as compared to other methods of heating.

The intermediate products according to the present invention are suitable to use for preparing heterocyclic compounds by reaction with dinucleophiles and in the following Figure 2 examples of heterocyclic compounds prepared by reaction of intermediate products according to the present invention, 3-dimethylamino propenoates, with different dinucleophiles are illustrated.

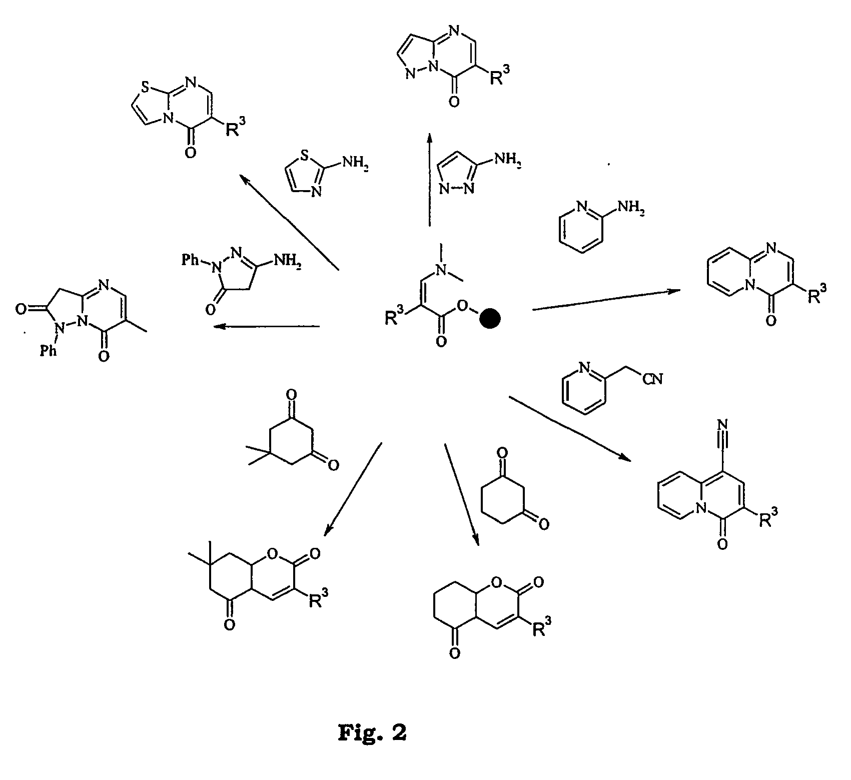

In the following Figure 3 examples of heterocyclic compounds prepared by reaction of intermediate products according to the present invention, 3-dimethylamino propenones, with different dinucleophiles are illustrated.

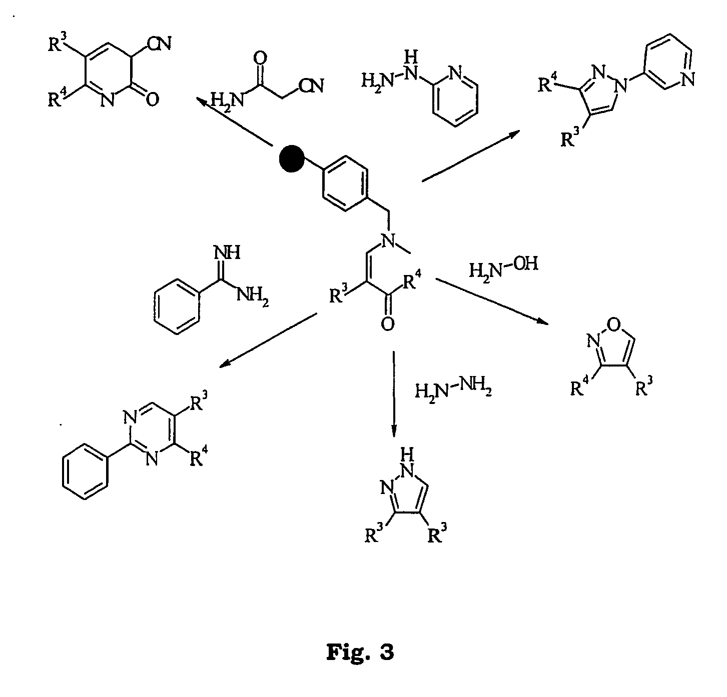

The solid polymeric support to which the compounds are coupled could be the following: a.) beads, pellets, disks, fibers, gels, or particles such as cellulose beads, pre-glass beads, silica gels, polypropylene beads, polyacrylamide beads, polystyrene beads that are lightly cross-linked with 1-2% divinylbenzene and optionally grafted with polyethylene glycol and optionally functionalized with amino, hydroxy, carboxy or halo groups; and b.) soluble supports such as low molecular weight non-cross-linked polystyrene and polyethylene glycol. The term solid support is used interchangeably with the term resin or bead in this invention and is intended to mean the same thing. For *propenoates* any resin which can form an alkyl or benzylic ester from a carboxylic acid such as polystyrene-divinylbenzene resins eg. Merrifield resin (benzyl chloride handle), Wang resin (benzyl alcohol handle), Tentagel PHB (benzyl alcohol handle), Resin with PAM anchor, Rink resin, PEGA resin are examples of suitable resins. For *propenones* any resin with a dialkyl amine handle, any resin which can form a dialkyl amine or benzyl alkyl amine handle such as Merrifield resin (displacement of a chloride with an alkylamine), Rink resin (methylation of a primary amine with methyliodide), amino methyl resin, trisamine resin, SASRIN resin, Behring resin, PAM resin, N-methylaminomethyl resin HCl are examples of suitable resins for use as solid support.

In the methods for preparing the intermediates according to the present invention N-disubstituted carboxamide acetals of the general formula **II** are suitable to use as a reactant 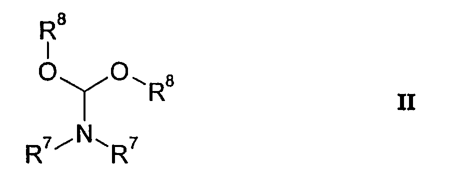 wherein the two R⁷ groups are the same or different groups selected from alkyl groups with 1 to 6 carbon atoms, cycloalkyl groups with 3 to 6 carbon atoms, benzyl groups, cyclic compounds including heterocyclic compounds with one or more heteroatoms, R⁷ is suitably methyl, ethyl. The two R⁷ groups could together be part of a carbocyclic or heterocyclic ring, for example imidazole.
The two R⁸ groups are the same or different groups selected from straight, branched or cyclic alkyl chains, substituted such alkyl chains, benzyl groups . R⁸ is suitably Me, Et. The two R⁸ groups could together be part of a carbocyclic ring for example 1,3 dioxane.

In a method of preparing an intermediate of formula I, which is coupled to the solid polymeric support through one or both R¹ groups (propenones) a polymeric support with a reactive secondary amine is prepared according to the following reaction whereby a product of formula IV is obtained 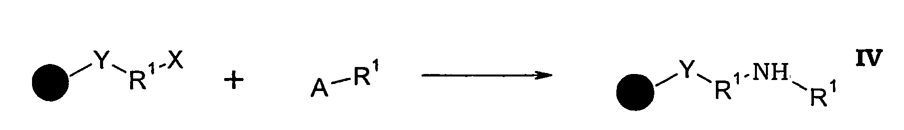 wherein Y is a spacer group which can be alkyl, benzyl, trityl or [OCH₂CH₂]ₙ, R¹ is as defined in claim 1, X is NH₂, halogen or triflate. When X is NH₂, A is halogen or triflate, and when X is halogen or triflate, A is NH₂, which product of formula IV then is reacted with a N-substituted carboxamide acetal of the general formula II as defined above
and a substance with a methylene or methyl group adjacent to a keto function according to the general formula III 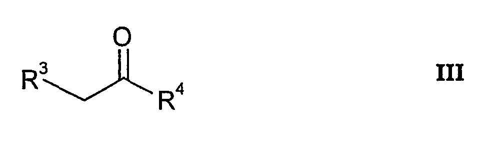 where R³, R⁴ are as defined in claim 1, whereby a compound of formula I coupled to the polymeric support through one or both of the R¹ groups is obtained.

In another method of preparing an intermediate of formula I, which is coupled to the solid polymeric support through one or both of the R¹ groups (propenones), a polymeric support with a reactive secondary amine is prepared according to the following reaction, whereby a product of formula V is obtained 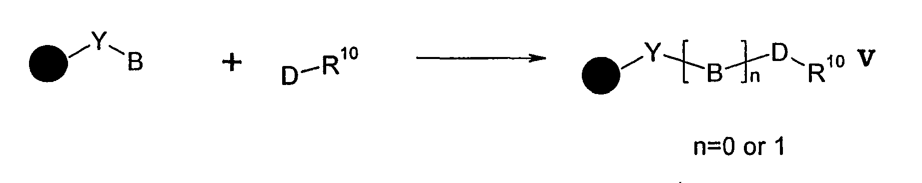 wherein Y is as defined above, R¹⁰ is a secondary amine, B and D are functional groups which form a covalent bond when reacted with each other, and when n=0, B is a leaving group, which product of formula V then is reacted with a N-disubstituted carboxamide acetal of the general formula II as defined above
and a substance with a methylene or methyl group adjacent to a keto function according to the general formula III as defined above, whereby a compound of formula I coupled to the polymeric support through one or both of the R¹ groups is obtained.

In the above methods for preparing the intermediates according to the present invention it is suitable to carry out the reactions by means of heating. The heating is suitably induced by the use of microwaves. The temperature is generally between 100 and 250°C. It is often suitable to carry out the reactions in a closed vessel.

The intermediate products according to the present invention are useful for the synthesis of heterocyclic compounds wherein a solid supported intermediate compound of the general formula I is reacted with a dinucleophile, i.e. a substance with two nucleophilic atoms selected from N, C, O and S adjacent to each other or separated by one or more carbon atoms, by heating the intermediate compound and the dinucleophilic substance in a solution for a short period of time which, after evaporation of the solvent, produces the desired heterocyclic compound in high yield and high purity.

The heating is suitably induced by the use of microwaves and a useful temperature is between 100 and 250°C. The reaction time needed is short and suitably less than 30 minutes.

*Dialkylamino propenoates* coupled to a solid support react with dinucleophiles in a two-step reaction wherein substitution of the dialkylamino group is followed by a nucleophilic attack on the ester functionality, which cleaves the ester.

*Dialkylamino propenones* coupled to a solid support react in a somewhat different manner with dinucleophiles. A condensation reaction with the keto function is followed by the substitution of the dialkylamino group.

*Heterocycle formation from dialkylamino propenones and dialkylamino propenoates.* The intermediates could be reacted with many dinucleophiles such as hydrazines, amidines, diketo substrates and 2-amino-pyridines to form heterocyclic compounds such as isoxazoles, pyrazoles, chromones, pyrimidines, pyranones, pyrimidones , pyranones, pyrimidones and substituted 4*H-*quinolizin-4-ones all of which have great interest as potential druglike compounds. Many of the mentioned products have in the literature been described to induce biological activity.
Dinucleophiles are defined as any substances with two nucleophilic atoms adjacent to each other or separated by one or more carbon atoms. The nucleophilic atoms are chosen from N, C, O, S. Examples of suitable dinucleophiles are hydroxylamine, hydrazine, substituted hydrazines, substituted amidines, 2-aminopyridines, 2-pyridino acetonitrile, 2-aminopyrazoline, 2-aminopyridazine, substituted 1,3-diketohexane and cyclohexane, 2-aminothiazole and 3-amino-2-pyraxolin-5-ones. Examples of some dinucleophiles are illustrated below.

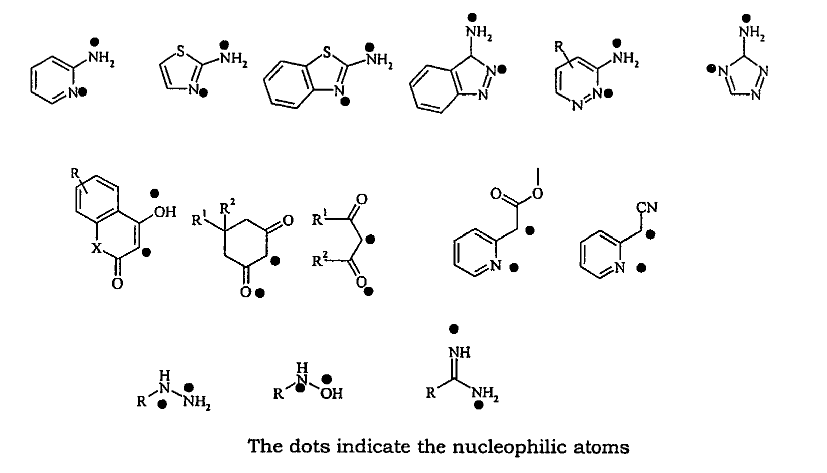

### Synthesis of solid phase bound dialkylamino propenoates

Formation of an intermediate product wherein the substrate is bound to the solid resin could be carried out in a two-step reaction, an example of which is shown in Fig. 4.

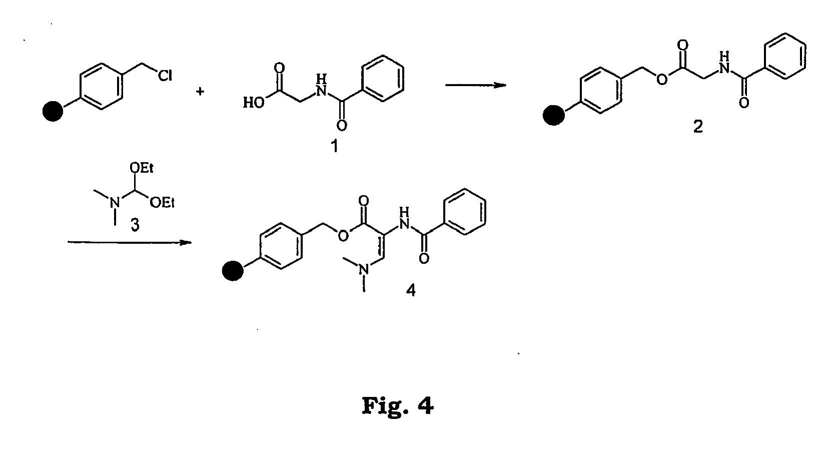

Fig. 4 shows the reaction between a solid support, Merrifield resin, and hippuric acid (1) in the first step and reaction of the product formed (2) with DMFDEA (3) which gives the intermediate product (4). Magic angle spinning NMR (MAS-NMR) analysis (Wehler, T.; Westman, J. *Tetrahedron Lett.* **1996**, *37*, 4771-4774) was used for the protocol development. An ester linkage was formed between the carboxylic acid substrate and the solid phase resin in step one. By comparing the peak area from the methylene group in the resin handle (PhCH₂Cl) and the peak area from the solid phase benzylester methylene group (PhCH₂OCO) the yield could be determined. In this example the Merrifield resin was treated with the N-acylated glycine derivative (hippuric acid) together with cesium carbonate in DMF under microwave heating at 200°C for 10 minutes. MAS-NMR analysis and elemental analysis showed a loading of approximately 1 mmol/g (80% yield), which is in the same range as described in the literature but in approximately a 100-fold shorter reaction time. Merrifield resin was suitable to use due to the high loading capacity and high thermal stability. After washing the resin was mixed with 5 eq. DMFDEA in 2.5 ml DMF and exposed to microwaves at 180°C for 10 minutes to form the dimethylamino propenoate intermediate product 4 according to the invention.

### Synthesis of solid phase bound dialkylamino propenones

An example of a synthesis method for preparing solid phase bound dialkylamino propenones is illustrated in Fig. 5.

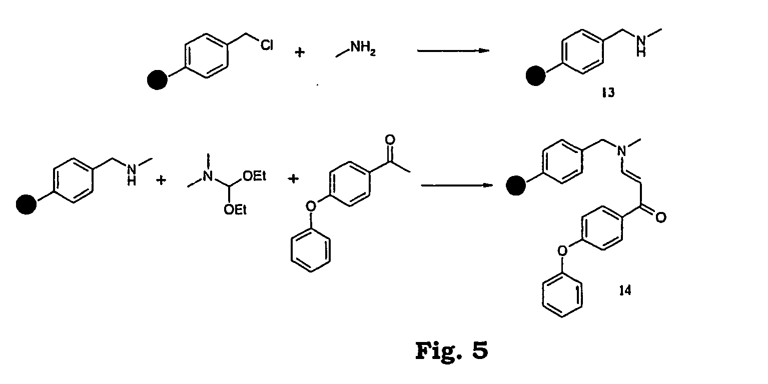

In the first step in this synthesis Merrifield resin was treated with methyl amine in water under microwave heating at 150°C for 10 minutes to form a benzyl methyl amine on the solid resin (13). After washing the resin was treated with 5 eq. DMFDEA together with 5 eq. 4-phenoxyacetophenone under microwave heating at 180°C for 10 minutes in DMF to form the intermediate product (14), the solid supported benzyl methyl aminopropenone according to the present invention in a three-component reaction.

The intermediate products according to the present invention were then used for the synthesis of heterocycles by reacting the solid supported intermediates with dinucleophiles in a suitable solvent e.g. at 180°C for 10 min.

In the following Fig. 6 examples of synthesis of heterocycles via solid phase bound dialkylamino propenoates are illustrated.

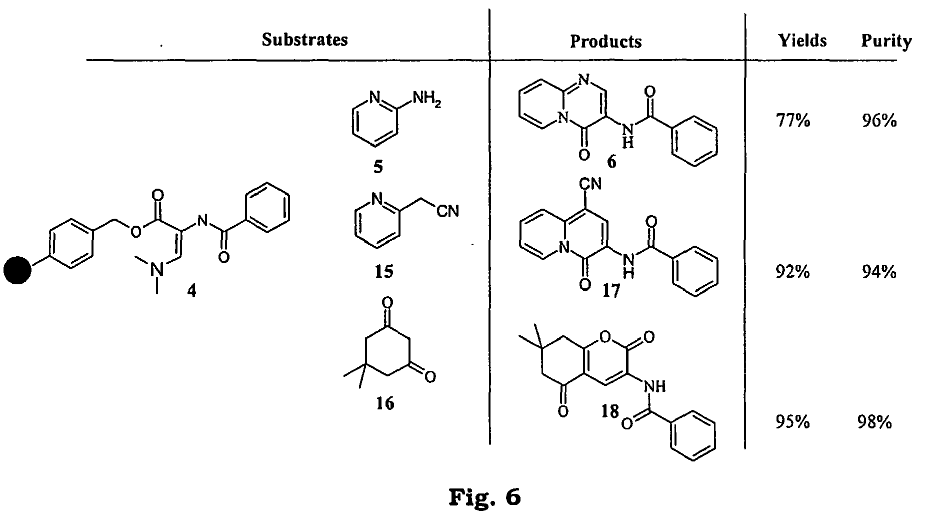

In the following Fig. 7 examples of synthesis of heterocycles via solid phase bound dialkylamino propenones are illustrated.

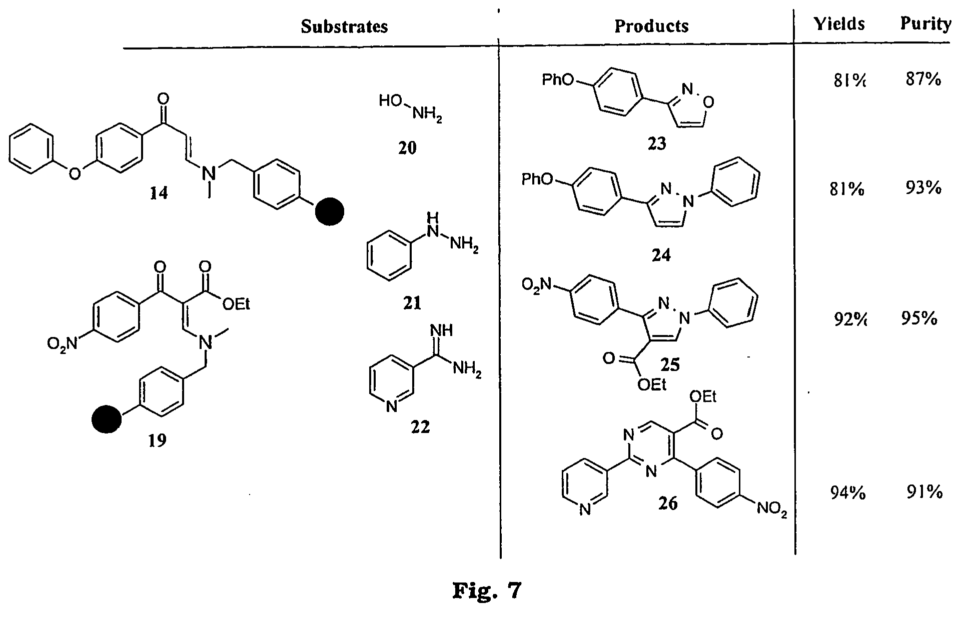

The invention is illustrated by means of the following examples, which are presented only for illustrative purpose and are not meant to limit the scope of the invention in any way.

### EXAMPLES

The microwave-assisted reactions were performed in a single mode microwave cavity, an instrument from Personal Chemistry. NMR spectra were recorded in CDCl₃ or DMSO-*d*₆ at 25°C, using a Bruker at 300 MHz (¹H)/75 MHz (¹³C) or a Varian 600 MHz instrument with a Nano probe for the MAS-NMR analysis. All NMR spectra recorded were in agreement with the postulated structures and only selected data are reported. Elemental analyses were performed by Mikrokemi AB, Uppsala, Sweden. All starting reagents were of the best grade available (Aldrich or Lancaster) and were used without purification. The reactions were run in a closed vessel and that in several cases the pressure during the reaction was between 5-20 bar.

***Coupling of N-benzoyl glycine* to *Merrifield resin (2)*.** 200 mg Merrifield resin (1.25 mmol/g loading capacity) was swelled in 2.5 mL DMF, 1.25 mmol (5 equiv.) *N-*benzoyl glycine (Hippuric acid) (**1**) and 1.25 mmol Cs₂CO₃ were added and the reaction mixture was heated at 200 °C for 10 min. The reaction mixture was then cooled down to room temperature by pressurized air. The residue was then washed several times with DMF, water and DCM. The resin was dried under reduced pressure in a desiccator. MAS-NMR analysis indicated compound **2** in a yield of 80% (approx. 1.0 mmol/g loading). 1H NMR (CDCl3): δ 4.24 (CO*CH*_{*2*}NCO), 5.11 (Ph*CH*_{*2*}CO), 7.3-7.4 (4H, aromatic), 7.8 (1H, aromatic). Elemental analysis: 1.35 weight percent giving 0.96 mmol/g loading.

***Methylamination of Merrifield resin (13)***. 200 mg Merrifield resin (1.25 mmol/g loading capacity) was treated with 2.0 mL methylamine in water (40% w/w) (excess) at 150 °C for 5 min. The reaction mixture was then cooled to room temperature by pressurized air. The residue was washed several times with Water, DCM and MeOH to give compound **13**. Elemental analysis gave 1.52 weight percent giving approx. 1.08 mmol/g loading.

***Dimethyl amino propenoates from N-benzoyl glycine on solid support (4).*** 250 mg of solid supported *N*-benzoyl glycine benzyl ester 2 (approximately 0.25 mmol) was swelled in 2.5 mL DMF, 1.57 mmol DMFDEA was added and the reaction mixture was heated at 180 °C for 10 min. The reaction mixture was then cooled to room temperature by pressurized air. The residue was washed several times with DMF, water and DCM. The resin was dried under reduced pressure in a desiccator. MAS-NMR analysis indicate compound **4** but no yield was determined due to low resolution.

***Benzyl methyl amino propenones from 4-phenoxy acetophenone* on** ***solid support (14).*** 200 mg of benzyl methylamine on solid support 13 (approximately 0.2 mmol) was swelled in 2.0 mL DMF, 214 µL DMFDEA and 155 µL 4-phenoxy acetophenone were added and the reaction mixture was heated at 180 °C for 10 min. The reaction mixture was then cooled down to room temperature by pressurized air. The residue was washed several times with DMF, water and DCM. The resin was dried under reduced pressure in a desiccator. MAS-NMR analysis indicated compound **14** but no yield was determined due to low resolution.

***Benzyl methyl amino propenones from ethyl 4-nitrobenzoylacetate on solid support (19)**.* 200 mg of Benzyl methylamine on solid support **13** was treated with ethyl 4-nitrobenzoylacetate as described above for the synthesis of compound **19**. MAS-NMR analysis indicated compound **19** but no yield was determined due to low resolution.

***3-(benzoyl)amino-4H-pyrido[1,2-a]pyrimidin-4-one (6.)*** 100 mg of the solid supported compound **4** were added to 6.6 mg 2-aminopyridine (5) (0.07 mmol) in 0.5 mL of acetic acid. The solution was exposed to microwaves at 180°C for 10 minutes and then cooled to room temperature. The acetic acid was evaporated giving 14.2 mg of product **6**, a total yield of 77% and 96% purity based on LC/MS analysis. The structure was confirmed by ¹H NMR (300 MHz, CDCl₃): δ 7.16 (dt, 1H, ArH), 7.45-7.65 (m, 4H, ArH), 7,75 (dd, 1H, ArH), 7.95 (dd, 2H, ArH), 8.84 (s, 1H, *N*H), 8.95 (dd, 1H, ArH), 9.75 (s, 1H, pyrimidin-H).

***3-(benzoyl)amino-1-cyano-4H-quinolizin-4-one (17)*.** 100 mg of the solid supported compound 4 were added to 5.6 µL 2-pyridyl-acetonitrile (15) (0.05 mmol) in 0.5 mL of acetic acid. The solution was exposed to microwaves at 180°C for 10 minutes and then cooled to room temperature. The acetic acid was evaporated. The residue was dissolved in DCM and filtered through a plug of silica. Crude analysis showed a LC/MS purity of 94%. Evaporation of the solvent gave the product **17** in 13.3 mg, a total yield of 92%. The structure was confirmed by ¹H NMR (300 MHz, CDCl₃): δ NMR 7.02 (ddd, 1H, ArH), 7.32-7.42 (m, 4H, PhH), 7.77 (m, 2H, ArH), 7.81 (dt, 1H, ArH), 8.88 (s, 1H, *N*H), 8.92 (dt, 1H, ArH), 9.11 (s, 1H, quinolizin-4-one).

***3-(benzoyl)amino-5-oxo-5,6,7,8-tetrahydro-2H-1-benzopyran-2-one* (18).** 100 mg of the solid supported compound **4** were added to 7.0 mg 5,5 dimethyl-1,3-cyclohexanedione (**16**) in 0.5 mL of acetic acid. The solution was exposed to microwaves at 180°C for 10 min. and then cooled to room temperature. The acetic acid was evaporated giving 14.8 mg of product **18**, a total yield of 95% and 98% purity based on LC/MS analysis. The structure was confirmed by ¹H NMR (300 MHz, CDCl₃): δ 1.2 (s, 6H, CH₃) 2.48 (s, 2H, CH₂), 2.78 (s, 2H, CH₂), 7.5-7.7 (m, 3H, ArH), 7.92 (m, 2H, ArH), 8.59 (s, 1H, *N*H), 8.83 (s, 1H, CH).

***(4-phenoxy)phenylisoxazole (23)*.** 200 mg of the solid supported compound **14** was mixed with 0.1 (0.5 equiv.) mmol of hydroxylamine hydrochloride (**20**) and 2 mL of EtOH. The mixture was exposed to microwaves at 180 °C for 10 minutes and then cooled to room temperature. The solvent was evaporated. The product 23 was isolated in 81% yield and 87% purity based on LC/MS analysis and characterized by ¹H NMR (300 MHz, CDCl₃): δ 6.44 (d, 1H, *J*=1.9 Hz, isoxazole), 7.04 (m, 4H, ArH), 7.17 (dt, 1H, ArH), 7.38 (m, 2H, ArH), 7.76 (m, 2H, ArH), 8.25 (d, 1H, J=1.9 Hz, isoxazole).

***1-phenyl-5-(4-phenoxyphenyl)-pyrazole (24)*.** 200 mg of the solid supported compound **14** was mixed with 0.1 (0.5 equiv.) mmol of phenylhydrazine (21) and 2 mL of acetic acid. The mixture was exposed to microwaves at 180 °C for 10 minutes and then cooled to room temperature. The solvent was evaporated. The product **24** was isolated in 81% yield and 93% purity and characterized by ¹H NMR (300 MHz, CDCl₃): δ 6.48 (d, 1H, *J*=1.9 Hz, pyrazole), 6.91 (dd, 2H, ArH), 7.03 (m, 2H, ArH), 7.18 (dd, 2H, ArH), 7.3-7.4 (m, 8H, ArH), 7.71 (d, 1H, *J*=1.9 Hz, pyrazole).

***Ethyl(1-phenyl-3-(4-nitro)-phenyl* p*yrazole-4-carboxylate (25).*** 200 mg of the solid supported compound **19** were treated as described for compound **24** using EtOH as solvent. The EtOH was evaporated giving 31.0 mg of product **25** in 92% yield and 91% purity. The structure was confirmed by ¹H NMR (300 MHz, CDCl₃): δ 1.28 (t, 3H, CH₃*CH*_{*2*}), 4.25 (q, 2H, CH₂*CH*_{*3*}), 7.20 (m, 2H, ArH), 7.35 (m, 3H, ArH), 7.51 (d, 2H, ArH), 8.21 (d, 2H, ArH), 8.23 (s, 1H, pyrazole).

***Ethyl 2-(4-pyridyl)-4-(4-nitrophenyl)-pyrimidine-5-carboxylate (26).*** 200 mg of the solid supported compound **19** in 2 mL DMF was treated with 0.1 mmol (approx. 0.5 equiv.) 4-amidinopyridine hydrochloride (**22**) and 0.15 mmol KOH, exposed to microwaves at 180 °C for 10 minutes and then cooled to room temperature. The solvent was evaporated. The product (26) was isolated in 94% (32.7 mg) in 91% purity. The structure was characterized by ¹H NMR (300 MHz, CDCl₃): δ 1.22 (t, 3H, CH₃*CH*_{*2*}), 4.28 (q, 2H, CH₂*CH*_{*3*}), 7.54 (m, 3H, PhH), 7.85 (m, 2H, PhH), 8.35 (dd, 2H, pyridyl), 8.56 (dd, 2H, pyridyl), 9.31 (s, 1H, pyrimidine).

According to the present invention activated aminopropenoates and aminopropenones on solid phase are provided which intermediate products can be used in combinatorial syntheses of a large number of different heterocycles with an overall reaction time of approximately 30 minutes to give the products in high purity in high to excellent yields. One major benefit obtained by this approach is that purification is not needed.

## Claims

1. An intermediate product suitable for synthesis of heterocyclic compounds which is a compound of the general formula I 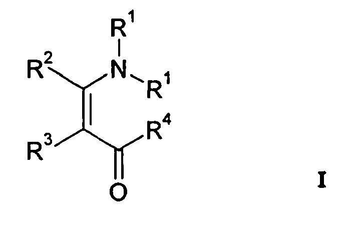 coupled to a solid polymeric support through one or both of the R¹ groups or through the R⁴ group
*wherein when coupled to the polymeric support through R*^{*1*}
the R¹ groups represent the same or different groups chosen from lower alkyl with 1 to 6 carbon atoms, such as methyl, ethyl , cycloalkyl with 3 to 6 carbon atoms in the ring, such as cyclopentyl, cyclohexyl, heterocyclic compounds including one or more heteroatoms, benzyl groups; two R¹ groups together can be included in heterocyclic ring containing one or more nitrogen atoms ;
R² represents H or a lower alkyl with 1 to 6 carbon atoms, such as methyl;
R⁴ represents unsubstituted or substituted aromatic ring(s), unsubstituted or substituted heteroaromatic ring(s) with one or more heteroatoms, or OR⁵;
when R⁴ is unsubstituted or substituted aromatic ring(s), unsubstituted or substituted heteroaromatic ring(s), R³ represents H, alkyl, unsubstituted or substituted aromatic ring, unsubstituted or substituted heteroaromatic ring with one or more heteroatoms, or COOR⁵;
when R⁴ is OR⁵, R³ is CN, COOR⁵, NCOR⁵, NCOOR⁵ or COR⁵;
the R⁵ groups, which can be the same or different, represent H, alkyl, benzyl, unsubstituted or substituted aromatic ring(s), unsubstituted or substituted heteroaromatic ring(s) with one or more heteroatoms;
*and wherein when coupled to the polymeric support through R*^{*4*}
R¹ and R² are as defined above,
R³ is as defined above except COOR⁵ and COR⁵
R⁴=OR⁵ where R⁵ is as defined above except H.

2. A method of preparing an intermediate of formula I according to claim 1, which is coupled to the solid polymeric support through one or both R¹ groups (propenones), wherein a polymeric support with a reactive secondary amine, is prepared according to the following reaction whereby a product of formula IV is obtained 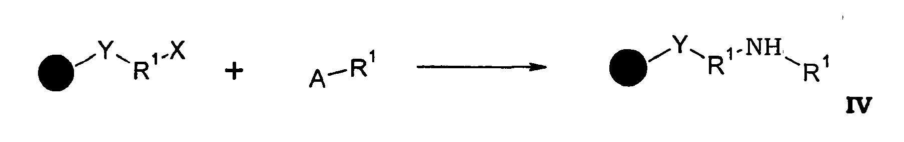 wherein Y is a spacer group which can be alkyl, benzyl, trityl or [OCH2CH2]ₙ, R¹ is as defined in claim 1, X is NH₂, halogen or triflate; when X is NH₂, A is halogen or triflate, and when X is halogen or triflate, A is NH₂, which product of formula IV then is reacted with a *N*-disubstituted carboxamide acetal of the general formula II 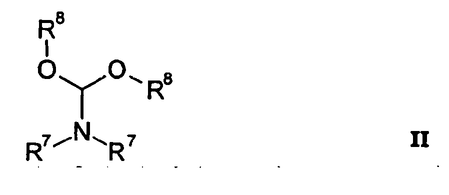 wherein the R⁷ groups are different or the same chosen from C1-C6 alkyl, cycloalkyl with 3 to 6 carbon atoms , benzyl groups, cyclic compounds including heteroatoms,the two R⁷ groups could together be part of a carbocyclic or heterocyclic ring,
the R⁸ groups could be the same or different chosen from straight, branched or cyclic alkyl chains, benzyl groups, and alkyl chains with substituents; the two R8 groups could together be part of a carbocyclic ring
and a substance with a methylene or methyl group adjacent to a keto function according to the general formula III 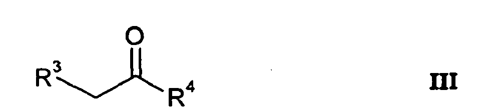 where R³, R⁴ are as defined in claim 1, whereby a compound of formula I coupled to the polymeric support through one or both of the R¹ groups is obtained.

3. A method of preparing an intermediate of formula I according to claim 1, which is coupled to the solid polymeric support through one or both R¹ groups (propenones), wherein a polymeric support with a reactive secondary amine is prepared according to the following reaction whereby a product of formula V is obtained 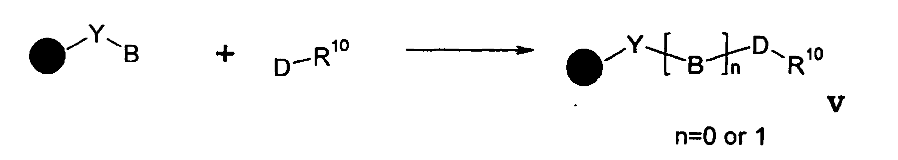 wherein Y is as defined in claim 2, R¹⁰ is a secondary amine, B and D are functional groups which form a covalent bond when reacted with each other, and when n=0 B is a leaving group,
which product of formula V then is reacted with a N-disubstituted carboxamide acetal of the general formula II 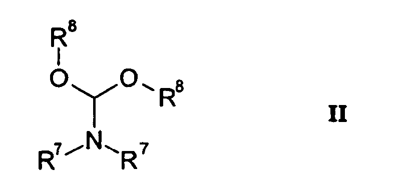 wherein R⁷ and R⁸ are defined as in claim 2,
and a substance with a methylene or methyl group adjacent to a keto function according to the general formula III 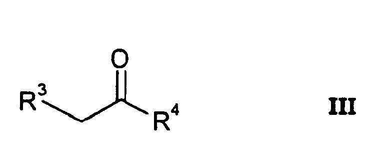 where R³, R⁴ are as defined in claim 1 whereby a compound of formula I coupled to the polymeric support through one or both of the R¹ groups is obtained.

4. Use of an intermediate product for synthesis of heterocyclic compounds which is a compound of the general formula **I** 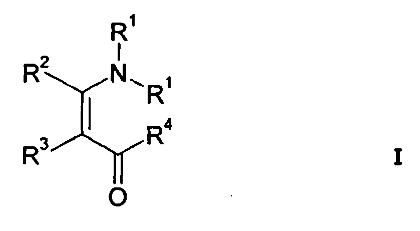 coupled to a solid polymeric support through one or both of the R¹ groups or through the R⁴ group,
*wherein when coupled to the polymeric support through R*^{*1*}*,*
the R¹ groups represent the same or different groups chosen from lower alkyl with 1 to 6 carbon atoms, such as methyl, ethyl, cycloalkyl with 3 to 6 carbon atoms in the ring, such as cyclopentyl, cyclohexyl, heterocyclic compounds including one or more heteroatoms, benzyl groups; two R¹ groups together can be included in a heterocyclic ring containing one or more nitrogen atoms ;
R² represents H or a lower alkyl with 1 to 6 carbon atoms, such as methyl;
R⁴ represents unsubstituted or substituted aromatic ring(s), unsubstituted or substituted heteroaromatic ring(s) with one or more heteroatoms or OR⁵;
when R⁴ is unsubstituted or substituted aromatic ring(s), unsubstituted or substituted heteroaromatic ring(s), R³ represents H, alkyl, unsubstituted or substituted aromatic ring, unsubstituted or substituted heteroaromatic ring with one or more heteroatoms or COOR⁵;
when R⁴ is OR⁵, R³ is CN, COOR⁵, NCOR⁵, NCOOR⁵ or COR⁵;
the R⁵ groups, which can be the same or different, represent H, alkyl, benzyl, unsubstituted or substituted aromatic ring(s), unsubstituted or substituted heteroaromatic ring(s) with one or more heteroatoms;
*and wherein when coupled to the polymeric support through R*^{*4*}
R¹ and R² are as defined above,
R³ is as defined above except COOR⁵ and COR⁵,
R⁴=OR⁵ where R⁵ is as defined above except H,
wherein the solid supported intermediate compound of the general formula **I** is reacted with a dinucleophile, i.e. a substance with two nucleophilic atoms selected from N,C,O and S adjacent to each other or separated by one or more carbon atoms, by reacting the intermediate compound and the dinucleophilic substance for a short period of time which after evaporation of the solvent produces the desired heterocyclic compound in high yield and in high purity.

5. A method according to claim 2 or 3 wherein the reactions are performed under heating.

6. A method according to claim 5 wherein the heating is induced by the use of microwaves.

7. A method according to any of claims 2-3 and 5-6 where the solid polymeric support to which the compounds are coupled is polystyrene beads that are lightly cross-linked with 1-2% divinylbenzene and optionally grafted with polyethylene glycol.

8. A method according to claims 2 and 7 where the solid polymeric support is polystyrene beads which are functionalized with halogen and triflate or NH₂.

9. A method according to claims 3 and 7 where the polystyrene beads are functionalized with B as defined in formula V.

10. A method according to claims 7 where the polystyrene beads are functionalized with halogen or hydroxyl.

11. A method according to claim 2 or 3 where the substance with a methylene or methyl group adjacent to a keto function according to the general formula III is a substituted acetophenone or a beta ketoester.

12. A method according to any of claims 2-11 wherein the disubstituted carboxamide acetal of formula II is dimethylformamide diacetal.

## Patentansprüche

1. Zur Synthese von heterocyclischen Verbindungen geeignetes Zwischenprodukt, das eine Verbindung der allgemeinen Formel I ist 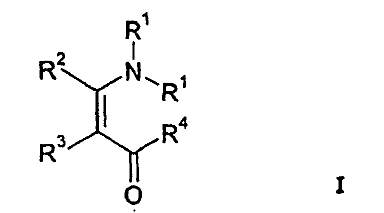 gekuppelt an einen festen polymeren Träger über eine oder beide R¹-Gruppen oder über die R⁴-Gruppe,
worin, wenn an den polymeren Träger über R¹ gekuppelte, bedeuten:
die R¹-Gruppen gleiche oder verschiedene Gruppen, ausgewählt aus Niederalkyl mit 1 bis 6 Kohlenstoffatomen, wie z.B. Methyl, Ethyl, Cycloalkyl mit 3 bis 6 Kohlenstoffatomen im Ring, wie z.B. Cyclopentyl, Cyclohexyl, heterocyclische Verbindungen, die ein oder mehrere Heteroatome aufweisen, Benzylgruppen; zwei R¹-Gruppen zusammen in einem ein oder mehrere Stickstoffatome enthaltenden heterocyclischen Ring enthalten sein können;
R² H oder ein Niederalkyl mit 1 bis 6 Kohlenstoffatomen, wie z.B. Methyl;
R⁴ unsubstituierten oder substituierten aromatischen Ring(e), unsubstituierten oder substituierten aromatischen heteroaromatischen Ring(e) mit einem oder mehreren Heteroatomen, oder OR⁵;
wenn R⁴ unsubstituierten oder substituierten aromatischen Ring(e), unsubstituierten oder substituierten heteroaromatischen Ring(e) bedeuten, R³ H, Alkyl, unsubstituierten oder substituierten aromatischen Ring, unsubstituierten oder substituierten aromatischen heteroaromatischen Ring mit einem oder mehreren Heteroatomen, oder COOR⁵ bedeutet;
wenn R⁴ OR⁵ ist, R³ CN, COOR⁵, NCOR⁵, NCOOR⁵ oder COR⁵ ist;
die R⁵-Gruppen, die gleich oder verschieden sein können, H, Alkyl, Benzyl, unsubstituierten oder substituierten aromatischen Ring(e), unsubstituierten oder substituierten heteroaromatischen Ring(e) mit einem oder mehreren Heteroatomen bedeutet, wenn an den polymeren Träger über R⁴ gekuppelt, bedeuten:
R¹ und R² die vorstehend angegebene Bedeutung,
R³ die vorstehend angegebene Bedeutung außer COOR⁵ und COR⁵,
R⁴ = OR⁵, worin R⁵ die vorstehend angegebene Bedeutung, außer H, aufweist.

2. Verfahren zur Herstellung eines Zwischenprodukts der Formel I nach Anspruch 1, das an den festen polymeren Träger über eine oder beide R¹-Gruppen gekuppelt ist (Propenone),
worin ein polymerer Träger mit einem reaktiven sekundären Amin nach der folgenden Reaktion hergestellt wird, wobei ein Produkt der Formel IV erhalten wird 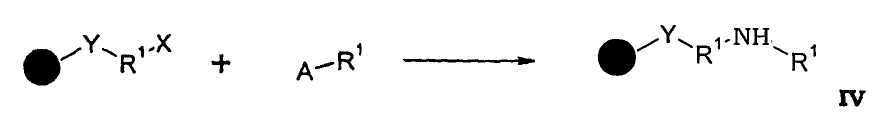 worin Y eine Abstandsgrupper ist, die Alkyl, Benzyl, Trityl oder [OCH₂CH₂]ₙ sein kann, R¹ die in Anspruch 1 angegebene Bedeutung besitzt, X NH₂, Halogen oder Triflat ist; wenn X NH₂ ist, ist A Halogen oder Triflat, und wenn X Halogen oder Triflat ist, ist A NH₂, und das Produkt der Formel IV dann mit einem N-disubstituierten Carboxamidacetal der allgemeinen Formel II umgesetzt wird 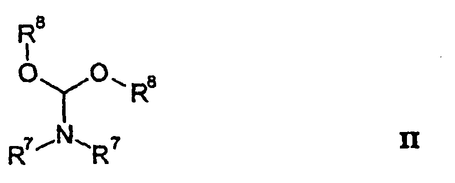 worin die R⁷-Gruppen verschieden oder gleich sind und ausgewählt sind aus C₁-C₆-Alkyl, Cycloalkyl mit 3 bis 6 Kohlenstoffatomen, Benzylgruppen, Heteroatome enthaltenden cyclischen Verbindungen, und die zwei R⁷-Gruppen zusammen Teil eines carbocyclischen oder heterocyclischen Rings sein können,
die R⁸-Gruppen gleich oder verschieden sein können und ausgewählt sind aus geradkettigen, verzweigten oder cyclischen Alkylketten, Benzylgruppen und Alkylketten mit Substituenten; die zwei R⁸-Gruppen zusammen Teil eines carbocyclischen Rings sein können,
und einer Substanz mit einer zu einer Keto-Funktion benachbarten Methylen- oder Methylgruppe der allgemeinen Formel III 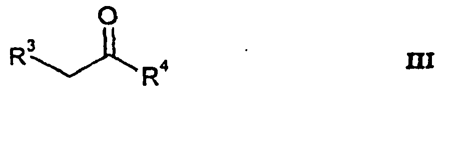 worin R³, R⁴ die in Anspruch 1 definierte Bedeutung aufweisen, wodurch eine an den polymeren Träger über eine oder beide der R¹-Gruppen gekuppelte Verbindung der Formel I erhalten wird.

3. Verfahren zur Herstellung eines Zwischenprodukts der Formel I nach Anspruch 1, das an den festen polymeren Träger über eine oder beide R¹-Gruppen gebunden ist (Propenone),
worin ein polymerer Träger mit einem reaktiven sekundären Amin nach der folgenden Reaktion hergestellt wird, wodurch ein Produkt der Formel V erhalten wird 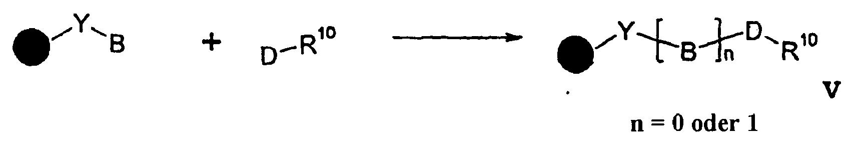 worin Y die in Anspruch 2 angegebene Bedeutung besitzt, R¹⁰ ein sekundäres Amin ist, B und D funktionelle Gruppen sind, die eine kovalente Bindung bilden, wenn sie mit einander reagieren, und wenn n = 0, B eine austretende Gruppe ist,
und das Produkt der Formel V dann mit einem N-substituierten Carboxamidacetal der allgemeinen Formel II umgesetzt wird 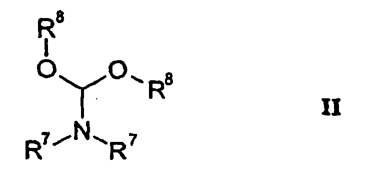 worin R⁷ und R⁸ die in Anspruch 2 angegebene Bedeutung aufweisen,
und einer Substanz mit einer zu einer Keto-Funktion benachbarten Methylen- oder Methylgruppe gemäß der allgemeinen Formel III 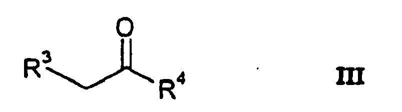 worin R³, R⁴ die an Anspruch 1 angegebene Bedeutung aufweisen, wodurch eine an den polymeren Träger über eine oder beide der R¹-Gruppen gekuppelte Verbindung der Formel I erhalten wird.

4. Verwendung eines Zwischenprodukts zur Synthese von heterocyclischen Verbindungen, das eine Verbindung der allgemeinen Formel I ist 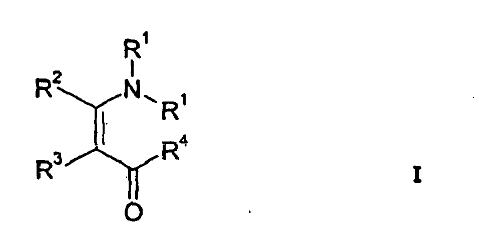 gekuppelt an einen festen polymeren Träger über eine oder beide R¹-Gruppen oder über die R⁴Gruppe,
worin, wenn an den polymeren Träger über R¹ gebunden, bedeuten:
die R¹-Gruppen gleiche oder verschiedene Gruppen ausgewählt aus Niederalkyl mit 1 bis 6 Kohlenstoffatomen, wie z.B. Methyl, Ethyl, Cycloalkyl mit 3 bis 6 Kohlenstoffatomen im Ring, wie z.B. Cyclopentyl, Cyclohexyl, heterocyclische Verbindungen, die ein oder mehreren Heteroatome enthalten, Benzylgruppen; zwei R¹-Gruppen zusammen in einem ein oder mehreren Stickstoffatome enthaltenden heterocyclischen Ring enthalten sein können;
R² H oder ein Niederalkyl mit 1 bis 6 Kohlenstoffatomen, wie z.B. Methyl;
R⁴ unsubstituierten oder substituierten aromatischen Ring(e), unsubstituierten oder substituierten heteroaromatischen Ring(e) mit einem oder mehreren Heteroatomen, oder OR⁵;
wenn R⁴ unsubstituierten oder substituierten aromatischen Ring(e), unsubstituierten oder substituierten heteroaromatischen Ring(e) bedeuten, R³ H, Alkyl, unsubstituierten oder substituierten aromatischen Ring, unsubstituierten oder substituierten heteroaromatischen Ring mit einem oder mehreren Heteroatomen, oder COOR⁵ bedeutet;
wenn R⁴ OR⁵ ist, R³ CN, COOR⁵, NCOR⁵, NCOOR⁵ oder COR⁵ ist;
die R⁵-Gruppen, die gleich oder verschieden sein können, H, Alkyl, Benzyl, unsubstituierten oder substituierten aromatischen Ring(e), unsubstituierten oder substituierten heteroaromatischen Ring(e) mit einem oder mehr Heteroatomen bedeuten;
und worin, wenn an den polymeren Träger über R⁴ gekuppelt, bedeuten:
R¹ und R² die vorstehend angegebene Bedeutung,
R³ die vorstehend angegebene Bedeutung außer COOR⁵ und COR⁵,
R⁴ = OR⁵, worin R⁵ die vorstehend angegebene Bedeutung außer H aufweist,
worin das an einen festen Träger gekuppelte Zwischenprodukt der allgemeinen Formel I mit einem Dinucleophil, d.h., einer Substanz mit zwei nucleophilen Atomen, ausgewählt aus N, C, O und S, benachbart zu einander oder durch eine oder mehreren Kohlenstoffatome von einander getrennt, umgesetzt wird durch Umsetzen des Zwischenproduktes und der dinucleophilen Substanz während eines kurzen Zeitraums, wodurch nach Verdampfen des Lösungsmittels die gewünschte heterocyclische Verbindung mit hoher Ausbeute und in hoher Reinheit erhalten wird.

5. Verfahren nach Anspruch 2 oder 3, worin die Umsetzungen unter Erhitzen durchgeführt werden.

6. Verfahren nach Anspruch 5, worin das Erhitzen durch Verwendung von Mikrowellen induziert wird.

7. Verfahren nach einem der Ansprüche 2 bis 3 und 5 bis 6, worin der feste polymere Träger, an den die Verbindungen gekuppelt sind, Polystyrol-Kügelchen bedeutet, die mit 1 bis 2% Vinylbenzol leicht vemetzt sind und gegebenenfalls mit Polyethylenglykol aufgepfropft sind.

8. Verfahren nach den Ansprüchen 2 und 7, worin der feste polymere Träger Polystyrol-Kügelchen bedeutet, die mit Halogen und Triflat oder NH₂ funktionalisiert sind.

9. Verfahren nach den Ansprüchen 3 und 7, worin die Polystyrol-Kügelchen mit wie in Formel V definiertem B funktionalisiert sind.

10. Verfahren nach Anspruch 7, worin die Polystyrol-Kügelchen mit Halogen oder Hydroxyl funktionalisiert sind.

11. Verfahren nach Anspruch 2 oder 3, worin die Substanz mit einer zur Keto-Funktion benachbarten Methylen- oder Methylgruppe gemäß der allgemeinen Formel III ein substituiertes Acetophenon oder ein β-Ketoester ist.

12. Verfahren nach einem der Ansprüche 2 bis 11, worin das disubstituierte Carboxamidacetal der Formel II Dimethylformamiddiacetal ist.

## Revendications

1. Produit intermédiaire approprié pour la synthèse de composés hétérocycliques, qui est un composé de formule générale I : 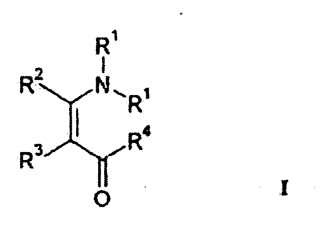 couplé à un support polymère solide par l'intermédiaire d'un ou des deux groupes R¹ ou par l'intermédiaire du groupe R⁴.
*dans lequel, lorsqu'il est couplé au support polymère par l'intermédiaire de R*^{*1*}*,*
les groupes R¹ désignent des groupes identiques ou des groupes différents sélectionnés parmi : un alkyle inférieur avec 1 à 6 atomes de carbone, tels que le méthyle ou l'éthyle, un cycloalkyle avec 3 à 6 atomes de carbone dans l'anneau, tels que le cyclopentyle, le cyclohexyle, des composés hétérocycliques comprenant un ou plusieurs hétéroatomes, des groupes benzyles; deux groupes R¹ peuvent être inclus dans un anneau hétérocyclique contenant un ou plusieurs atomes d'azote ;
R² désigne H ou un alkyle inférieur avec 1 à 6 atomes de carbone, tel que le méthyle ;
R⁴ désigne un ou plusieurs anneaux aromatiques non substitués ou substitués, un ou plusieurs anneaux hétéroaromatiques non substitués ou substitués avec un ou plusieurs hétéroatomes, ou OR⁵ ;
lorsque R⁴ désigne un ou plusieurs anneaux aromatiques non substitués ou substitués, un ou plusieurs anneaux hétéroaromatiques non substitués ou substitués, R³ désigne H, un alkyle, un anneau aromatique non substitué ou substitué, un anneau hétéroaromatique non substitué ou substitué avec un ou plusieurs hétéroatomes, ou COOR⁵;
lorsque R⁴ désigne OR⁵, R³ désigne CN, COOR⁵, NCOR⁵, NCOOR⁵ ou COR⁵;
les groupes R⁵, qui peuvent être identiques ou différents, désignent H, un alkyle, un benzyle, un ou plusieurs anneaux aromatiques non substitués ou substitués, un ou plusieurs anneaux hétéroaromatiques non substitués ou substitués avec un ou plusieurs hétéroatomes ;
*et dans lequel, lorsqu'il est couplé au support polymère par l'intermédiaire de R*^{*4*}*,*
R¹ et R² sont tels que définis ci-dessus
R³ est tel que défini ci-dessus, excepté COOR⁵ et COR⁵
R⁴ = OR⁵, R⁵ étant tel que défini ci-dessus, excepté H.

2. Procédé de préparation d'un intermédiaire de formule I selon la revendication 1, couplé au support polymère solide par l'intermédiaire d'un ou des deux groupes R¹ (propénones), dans lequel un support polymère avec un amine secondaire réactif est préparé selon la réaction suivante, moyennant quoi un produit de formule IV est obtenu 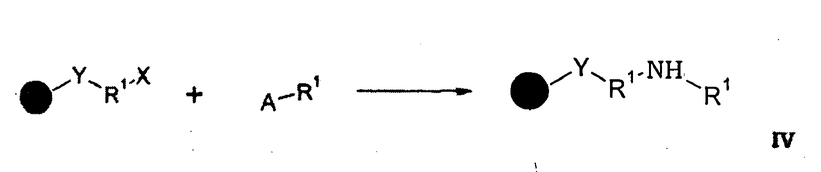 dans lequel Y est un groupe intercalaire pouvant être un alkyle, un benzyle, un trityle ou [OCH2CH2]ₙ, R¹ est tel que défini dans la revendication 1, X désigne NH₂, un halogène ou un triflate ; lorsque X désigne NH₂, A désigne un halogène ou un triflate, lorsque X désigne un halogène ou un triflate, A désigne un NH₂, le produit de formule IV étant ensuite mis en réaction avec un acétal carboxamide N-disubstitué présentant la formule générale II : 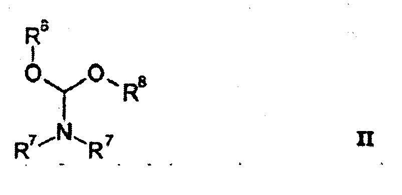 dans lequel les groupes R⁷ sont des groupes différents ou identiques sélectionnés parmi : un alkyle C1-C6, un cycloalkyle avec 3 à 6 atomes de carbone, des groupes benzyles, des composés cycliques comprenant des hétéroatomes, les groupes R⁷ pouvant également faire partie d'un anneau carbocyclique ou hétérocyclique,
dans lequel les groupes R⁸ peuvent être des groupes identiques ou différents sélectionnés parmi : des chaînes alkyles linéaires, ramifiées ou cycliques, des groupes benzyles et des chaînes alkyles avec des substituants ; les groupes R⁸ pouvant également faire partie d'un anneau carbocyclique,
et une substance avec un groupe méthylène ou méthyle adjacent à la fonction cétone, présentant la formule générale III : 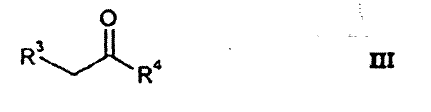 dans lequel R³ et R⁴ sont tels que définis dans la revendication 1, moyennant quoi un composé de formule I couplé au support polymère par l'intermédiaire d'un ou des deux groupes R¹ est obtenu.

3. Procédé de préparation d'un intermédiaire de formule I selon la revendication 1, couplé au support polymère par l'intermédiaire d'un ou des deux groupes R1 (propénones), dans lequel un support polymère avec un amine secondaire réactif est préparé selon la réaction suivante, moyennant quoi un produit de formule V est obtenu : 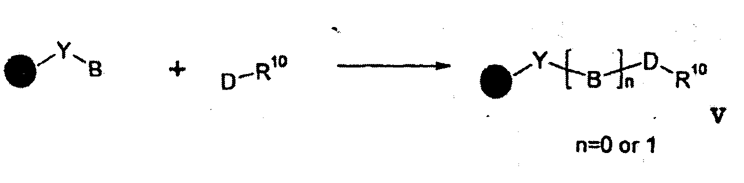 dans lequel Y est tel que défini dans la revendication 2, R¹⁰ est un amine secondaire, B et D sont des groupes fonctionnels qui forment une liaison covalente lorsqu'ils sont mis en réaction, l'un avec l'autre et lorsque n = 0, B est un groupe partant,
ce produit de formule V étant ensuite mis en réaction avec un acétal carboxamide N-disubstitué de formule générale II 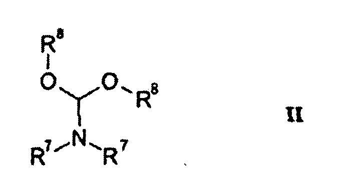 dans lequel R⁷ et R⁸ sont tels que définis dans la revendication 2,
et une substance présentant un groupe méthylène ou un groupe méthyle adjacent à une fonction cétone selon la formule générale III 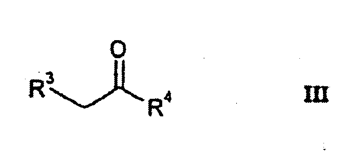 dans lequel R3 et R4 sont tels que définis dans la revendication 1, moyennant quoi un composé de formule I, couplé au support polymère par l'intermédiaire d'un ou des deux groupes R¹, est obtenu.

4. Utilisation d'un produit intermédiaire pour la synthèse de composés hétérocycliques qui est un composé de formule générale I : 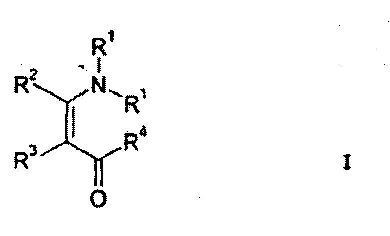 couplé à un support polymère solide par l'intermédiaire d'un ou des deux groupes R¹ ou par l'intermédiaire du groupe R⁴,
*dans lequel, lorsqu'il est couplé au support polymère par l'intermédiaire de R*^{*1*}*,*
les groupes R1 désignent des groupes identiques ou différents sélectionnés parmi : un alkyle inférieur avec 1 à 6 atomes de carbone, tels que le méthyle ou l'éthyle, un cycloalkyle avec 3 à 6 atomes de carbone dans l'anneau, tels que le cyclopentyle, le cyclohexyle, des composés hétérocycliques comprenant un ou plusieurs hétéroatomes, des groupes benzyles ; deux groupes R¹ peuvent être inclus dans un anneau hétérocyclique contenant un ou plusieurs atomes d'azote ;
R² désigne H ou un alkyle inférieur avec 1 à 6 atomes de carbone, tel que le méthyle ;
R⁴ désigne un ou plusieurs anneaux aromatiques non substitués ou substitués, un ou plusieurs anneaux hétéroaromatiques non substitués ou substitués avec un ou plusieurs hétéroatomes, ou OR⁵ ;
lorsque R⁴ désigne un ou plusieurs anneaux aromatiques non substitués ou substitués, un ou plusieurs anneaux hétéroaromatiques non substitués ou substitués, R³ désigne H, un alkyle, un anneau aromatique non substitué ou substitué, un anneau hétéroaromatique non substitué ou substitué avec un ou plusieurs hétéroatomes, ou COOR⁵ ;
lorsque R⁴ désigne OR⁵, R³ désigne CN, COOR⁵, NCOR⁵, NCOOR⁵ ou COR⁵;
les groupes R⁵, qui peuvent être identiques ou différents, désignent H, un alkyle, un benzyle, un ou plusieurs anneaux aromatiques non substitués ou substitués, un ou plusieurs anneaux hétéroaromatiques non substitués ou substitués avec un ou plusieurs hétéroatomes ;
*et dans lequel, lorsqu'il est couplé au support polymère par l'intermédiaire de R*^{*4*},
R¹ et R² sont tels que définis ci-dessus
R³ est te! que défini ci-dessus excepté COOR⁵ et COR⁵
R⁴ = OR⁵, R⁵ étant tel que défini ci-dessus excepté H,
dans lequel le composé intermédiaire à support solide de formule général I est mis en réaction avec un dinucléophile, c'est-à-dire une substance avec deux atomes nucléophiles sélectionnés parmi : N, C, O et S, adjacents les uns par rapport aux autres ou séparés par un ou plusieurs atomes de carbone, en mettant en réaction le composé intermédiaire et la substance dinucléophile pendant un court laps de temps, l'évaporation du solvant produisant le composé hétérocyclique approprié avec un fort rendement et une pureté élevée.

5. Procédé selon la revendication 2 ou 3, dans lequel les réactions sont effectuées sous l'action de la chaleur.

6. Procédé selon la revendication 5, dans lequel la chaleur est produite par des micro-ondes.

7. Procédé selon l'une des revendications 2-3 et 5-6, dans lequel le support polymère solide auquel les composés sont couplés est constitué de billes de polystyrène légèrement réticulées avec 1 à 2% de divinylbenzène et, en option, greffées avec du polyéthylène glycol.

8. Procédé selon les revendications 2 et 7, dans lequel le support polymère solide est constitué de billes de polystyrène fonctionnalisées avec un halogène et un triflate ou du NH₂.

9. Procédé selon les revendications 3 et 7, dans lequel les billes de polystyrène sont fonctionnalisées avec B tel qu'il est défini dans la formule V.

10. Procédé selon la revendication 7, dans lequel les billes de polystyrène sont fonctionnalisées avec un halogène ou un hydroxyle.

11. Procédé selon l'une des revendications 2 ou 3, dans lequel la substance avec un groupe méthylène ou un groupe méthyle adjacent à une fonction cétone selon la formule générale III est un acétophénone substitué ou un béta-cétone-ester.

12. Procédé selon l'une des revendications 2 à 11, dans lequel l'acétal carboxamide disubstitué de formule Il est du diacétal de diméthylformamide.
